# EUROPEAN PATENT APPLICATION

(11) **EP 4 763 139 A1**
(43) Date of publication of application: **24.06.2026**
(21) Application number: 25201965.8
(22) Date of filing: 12.09.2025
(51) Int. Cl.: A61F 2/24

(54) **STENT TAB RETAINERS**

(30) Priority: 20.12.2024 US 202463737414 P
(71) Applicant: St. Jude Medical, Cardiology Division, Inc., St. Paul, MN 55117 (US)
(72) Inventor: ZIOBRO, John, Centerville, 55038 (US); STEENWYK, Nicholas, Minneapolis, 55410 (US); BARRETTE, Alexander, Maplewood, 55109 (US); HIGH, Keith, White Bear Lake, 55110 (US); GOVEK, Tyler, Hopkins, 55343 (US); MORRISSEY, Michael Shane, St. Paul, 55116 (US)
(74) Representative: Gill Jennings & Every LLP

(57) **Abstract**

Aspects of the disclosure provide a delivery device for a prosthetic heart valve, the delivery device includes a retaining element with a recess arranged to receive a corresponding tab of the prosthetic heart valve. The recess may be shaped to progressively reduce contact with the respective tab as the prosthetic heart valve expands from its collapsed condition to its expanded condition. The recess may include a tapered base. The retaining element may include one or more secondary recesses arranged to receive one or more struts of the prosthetic heart valve. A prosthetic heart valve may also be provided, in which the tab is set at an angle directed away from a plane of struts connecting the tab and/or the tab may include a biasing element arranged to bias the tab away from the recess when the tab is in the recess.

## Description

### BACKGROUND

The present disclosure relates to a collapsible prosthetic heart valve and delivery systems for collapsible prosthetic heart valves.

Prosthetic heart valves that are collapsible to a relatively small circumferential size can be delivered into a patient less invasively than valves that are not collapsible. For example, a collapsible valve may be delivered into a patient via a tube-like delivery apparatus such as a catheter, a trocar, a laparoscopic instrument, or the like. This collapsibility can avoid the need for a more invasive procedure such as full open-chest, open-heart surgery.

Collapsible prosthetic heart valves typically take the form of a valve structure mounted on a stent. There are two types of stents on which the valve structures are ordinarily mounted: a self-expanding stent and a balloon-expandable stent. To place such valves into a delivery apparatus and ultimately into a patient, the valve must first be collapsed or crimped to reduce its circumferential size.

When a collapsed prosthetic valve has reached the desired implant site in the patient (e.g., at or near the annulus of the patient's heart valve that is to be replaced by the prosthetic valve), the prosthetic valve can be deployed or released from the delivery apparatus and expanded (or re-expanded) to full operating size. For balloon-expandable valves, this generally involves assuring its proper location, and then expanding a balloon positioned within the valve stent. For self-expanding valves, on the other hand, the stent automatically expands as a sheath covering the valve is withdrawn.

### SUMMARY OF THE DISCLOSURE

Various aspects of the present invention are set out in the appended claims.

According to aspects of the disclosure there may be provided a delivery device for a prosthetic heart valve, the delivery device comprising: an inner shaft; a distal sheath disposed about a portion of the inner shaft to form a compartment with the inner shaft, the compartment being sized to receive the prosthetic heart valve, the inner shaft and the distal sheath being movable relative to one another; and, a retaining element coupled to the inner shaft and defining an end of the compartment, the retaining element comprising a recess arranged to receive a corresponding tab of the prosthetic heart valve.

The retaining element may define a proximal end of the compartment. The compartment may be defined between an atraumatic tip coupled to the inner shaft and the retaining element, the retaining element forming a proximal end of the compartment. The distal sheath may be movable from a closed position fully covering the compartment and an open position uncovering the compartment.

The retaining element may comprise a plurality of recesses (pockets) arranged around the periphery of the retaining element each for receiving a respective tab of the prosthetic heart valve. For example, where the prosthetic heart valve includes three tabs the retaining element may comprise three corresponding recesses.

According to a first aspect of the disclosure, the recess may be shaped to progressively reduce contact with the respective tab as the prosthetic heart valve expands from its collapsed condition to its expanded condition. In this way, the release of the valve can be facilitated (e.g. interference with tab release by the recess can be reduced) while still securing the valve during delivery.

As such, one or more of the dimensions of the recess may increase from a base of the recess to the outer surface of the retaining element in which the recess is formed. Hence, the recess may comprise a larger width perpendicular to the insertion direction or a larger length parallel to the insertion direction at the surface of the retaining element than at the base of the recess.

In some implementations, the one or more outer edges of the recess may be profiled so that the one or more outer edges are angled outward. By profiled we mean that edges may be shaped or contoured to reduce contact as the tab releases. The term 'profiled' is meant to be used as a collective term used to describe any non-90-degree edge treatment. Removing material at the edge allows the tab to slide out of the recess (pocket) upon release more readily, especially in situations where the tab is being forced into the wall of the recess by certain anatomical scenarios.

In other words, the one or more outer edges of the recess may be angled outward from the base to the opening, creating a wider opening than at the base. The recess may have an opening and the one or more outer edges of the recess may be profiled to increase the dimensions of the recess at the opening relative to the dimensions of a portion of the recess arranged to receive the corresponding tab.

In one particular implementation, the recess may comprise one or more radiused outer edges. Radiused outer edges may comprise a rolled edge or other curved profile. A radiused edge facilitates release and may be configured to reduce contact in a non-linear manner so as to facilitate faster release as release is started but to ensure proper securement at the base of the curvature.

Preferably the recess comprises a distal opening, two opposing side walls and a proximal wall, the side walls comprising a straight portion and a distal neck portion, the one or more radiused outer edges are the edges of the distal neck portion. The distal neck portion may be a narrowing portion of the side wall reducing the width of the recess from the proximal wall to the opening. By radiusing the edges of the distal neck portion, the release of the tab is improved. Studies by the inventors have found that radiusing the neck and/or the side walls only may allow for improved release without negatively impacting the ability of the retainer to push the valve distally, for example, since distal pushing may be performed by the proximal wall.

Additionally, the entire outer edge of the recess is radiused. The outer edge may preferably be the outermost edge. By radiusing all the outermost edges, a consistent release profile may be ensured.

In an alternative implementation, the recess may comprise one or more tapered outer edges. The tapered outer edges may be chamfered. The taper may preferably be between 0 degrees and 45 degrees, more preferably between 0 degrees and 30 degrees. The tapered outer edges may be configured so as to remove material that impedes tab movement out of the recess. The added taper or chamfer allows the tab to slide out upon release with a linear contact force applied to the tab as it progresses. A taper may also be simply, easily and consistently machined that allows for consistency of manufacture which is essential in medical devices.

Additionally, the recess comprises one or more tapered walls. In other words, the taper or chamfer extends to the base of the recess. This ensures a smooth release profile as the tab moves away from the base of the recess upon which it sits after loading.

In this implementation, the recess may comprise a distal opening, two opposing side walls and a proximal wall, the side walls comprising a straight portion and a distal neck portion, wherein the distal neck portion is tapered. The distal neck portion may be a narrowing portion of the side wall reducing the width of the recess from the proximal wall to the opening. By tapering the edges of the distal neck portion, the release of the tab is improved since those portions have an oversized effect on the release, in contrast to those other walls contributing to axial and circumferential movement of the tab.

Further, the recess may comprise a tapered proximal wall. The proximal wall or rear wall of the recess may contribute to preventing axial movement or otherwise may not be axially load bearing depending on the configuration of the heart valve and retaining element. The proximal wall may be shaped separately and differently to the side walls as it has an impact only on axial movement as compared to rotational movement and may disproportionately affect valve release compared to its contribution in securing the valve.

According to a second aspect of the disclosure, the recess may comprise a tapered base. Adapting the base of the recess in this way allows the tapered tab to sit parallel to the base of the recess and reduces the amount of open space underneath tabbed stent struts which helps prevent non-tabbed struts from passing underneath the retaining element and causing misload.

Preferably, the tapered base may comprise a taper extending from a distal opening of the recess adjacent the prosthetic heart valve when the prosthetic heart valve is secured on the retaining element to a proximal wall of the recess remote from the prosthetic heart valve when the prosthetic heart valve is secured on the retaining element. Put another way, the depth of the recess may increase in a distal to proximal direction.

In certain implementations the tapered base comprises a taper configured to have an angle of taper corresponding to an angle of the tab of the prosthetic heart valve relative to a longitudinal axis of the prosthetic heart valve. Preferably, the tapered base comprises a taper configured to have an angle of taper corresponding to an angle of the tab of the prosthetic heart valve relative to a longitudinal axis of the prosthetic heart valve during delivery, e.g. when in the collapsed condition, when the tab of prosthetic heart valve is received in the recess or when the prosthetic heart valve is secured on the retaining element. Since the tapered base and the tab are substantially parallel in this implementation, this alleviates outward force on the tab by the recess and thereby reduces the occurrence of misalignment and misload.

Preferably the angle is between 0 degrees and 20 degrees. More preferably the angle is between 7 degrees and 10 degrees. The angle being defined relative to the horizontal, i.e. the axis of the retaining element. In some implementations the angle of taper may substantially match the angle of the respective tab.

According to a third aspect of the disclosure, the recess may be a primary recess and the retaining element may further comprise one or more secondary recesses arranged to receive one or more struts of the prosthetic heart valve. The secondary recesses may be arranged to prevent strut crossover.

Preferably, the one or more secondary recesses are arranged to receive a respective apex of struts of the prosthetic heart valve. More preferably the one or more secondary recesses are arranged to receive a respective apex of struts in an outflow row of struts of the prosthetic heart valve. A plurality of secondary recesses may be spaced out around the diameter of the retaining element (e.g. regularly spaced in a circumferential direction). In other words, the one or more secondary recesses are arranged to receive non-tabbed strut apices (e.g. the outflow end of the diamond cells which do not have tabs extending therefrom).

During loading, each of the stent apices, e.g. aortic stent apices, may be positioned in an individual secondary recess. The secondary recesses prevent the non-tabbed aortic struts from crossing over or under one another. If one of these stent struts crosses underneath a tabbed stent strut, it can prevent the tabbed stent strut from fully engaging with the retaining element, resulting in misload.

In optional implementations the one or more secondary recess are formed as an indentation in an outer diameter of the retaining element. This simple adaption to the retaining element outer diameter is thus easy to machine and replicable and allows the apices to sit neatly in the indentation without affecting release and without placing load onto the struts during delivery.

Any of the first, second and third aspects of the disclosure may be combined so as to provide an implementation with the respective features of both aspects so as to improve securement and release of the valve.

According to further aspects of the disclosure, there may be provided a prosthetic heart valve, comprising: a stent; a valve assembly supported by the stent; and a tab at one end of the stent arranged to sit in a recess of a retaining element of a delivery device during delivery.

According to a fourth aspect of the disclosure, the tab may be set at an angle directed away from a plane of struts connecting the tab, such that an angle between the tab and a base of the recess is less than an angle between the plane of struts connecting the tab and a plane of the base of the recess, when the tab is in the recess during delivery. The angle may be outward of the stent. This reduces the radial force between the tab and the recess, thus reducing misalignment and misload and preventing stresses on the tabs during loading and delivery.

In preferred implementations the angle may be configured such that the tab is aligned with a plane of the base of the retainer. Stent tab orientation may be modified to be aligned with the axis of the retaining element or delivery system rather than follow the natural curvature of the aortic cells. This reduces the amount that the tabs are radially driven into the retainer by the set shape of the Nitinol stent. The change in angle may also cause the tab to lie flat in the retainer recess which reduces the amount of open space under the tabbed stent struts which helps prevent non-tabbed struts from passing underneath it and causing misload.

Preferably the angle is between 0 degrees and 20 degrees relative to the plane of the of the struts of the outflow row of cells. Preferably the struts forming a row of struts at the outflow end. More preferably between 7 degrees and 10 degrees.

According to a fifth aspect of the disclosure, the tab comprises a biasing element arranged to bias the tab away from the recess when the tab is in the recess. The biasing element may be arranged to push the tab out of the recess as soon as the tab is freed from the capsule, improving release.

In certain implementations the biasing element is a shape set spring loaded element. The element may be shape set into a nitinol tab of the stent.

Preferably wherein the biasing element extends from a base of the biasing element in an inward direction away from the tab, the base coupled to the tab at an inflow end of the biasing element. That is, the biasing element extends in an inflow to outflow direction. Directing the biasing element inwardly in this way reduces impact on the blood flow through the valve.

More preferably the biasing element is formed by a cut out in the tab.

In one implementation, the biasing element is formed by a geometric shape cut into the tab. This allows for simple and reliable methods of manufacture that are resilient and do not negatively affect securement and valve operation.

Optionally, in this implementation the shape is an inverted v-shape extending in an outflow direction from a base coupled to the tab at its widest point.

Further the biasing element may be integral with the tab.

In a specific implementation the biasing element is formed by shape setting a portion of the tab at an inward angle relative to a plane of the tab, the portion of the tab comprising a free end and a junction with the tab, the free end being inward of the tab. The free end may be a free outflow end. This inwardly deflected portion acts as a spring to bias the tab away from the bottom surface of the retainer recess. The spring is compressed by the loading system during encapsulation, then pushes the tab out of the retainer recess as soon as the tab is freed from the capsule, improving release.

In certain particularly beneficial implementations, the biasing element is configured to be visible under fluoroscopy. This can help to confirm correct tab placement.

According to a further aspect of the disclosure there may also be provided a prosthetic heart valve delivery system comprising a delivery device according to any of the first to third aspects of the disclosure and a prosthetic heart valve according to any of the fourth of fifth aspects of the disclosure.

### BRIEF DESCRIPTION OF DRAWINGS

Embodiments in accordance with the present disclosure will now be described with reference to the accompanying drawings, in which:
Figure 1 is a perspective view of a distal portion of a known delivery device;
Figure 2 is a perspective view of a proximal portion of the delivery device of Figure 1;
Figure 3 is an enlarged side view of a retaining element of the delivery device shown in FIGS. 1 and 2;
Figure 4 is an enlarged alternative view of a retaining element of a delivery device;
Figure 5 is a side elevational view of a known collapsible prosthetic heart valve in an expanded condition, showing the valve assembly attached to the stent;
Figure 6A is a perspective view of a retaining element in accordance with an example of the present disclosure;
Figure 6B is a schematic cross-sectional view of Figure 6A;
Figure 7A is a perspective view of a retaining element in accordance with an example of the present disclosure;
Figure 7B is a schematic cross-sectional view of Figure 7A;
Figure 7C is an alternative of Figure 7B;
Figure 7D is an alternative of Figures 7B and C;
Figure 8 is a schematic side view of a stent of a collapsible prosthetic heart valve;
Figure 9A is a perspective view of a stent loaded on a retaining element in accordance with an example of the present disclosure;
Figure 9B is a schematic cross-sectional view of the retaining element of Figure 9A;
Figure 10 is a schematic partial of a collapsible prosthetic heart valve loaded on a delivery device;
Figure 11 is a perspective view of a stent loaded on a retaining element in accordance with an example of the present disclosure;
Figure 12 is an annotated partial view of a stent of a collapsible prosthetic heart valve;
Figure 13 a schematic cross-sectional partial view of a stent of a collapsible prosthetic heart valve in accordance with an example of the present disclosure;
Figure 14A is a schematic view of a tab in accordance with an example of the present disclosure; and,
Figure 14B is a schematic side view of the tab of Figure 14A.

### DETAILED DESCRIPTION

As used herein in connection with prosthetic heart valves, the term "inflow end" refers to the end of the heart valve through which blood first flows during antegrade blood flow through the prosthetic heart valve, whereas the term "outflow end" refers to the end of the heart valve through which blood last flows during antegrade blood flow through the prosthetic heart valve. When used in connection with devices for delivering a prosthetic heart valve into a patient, the terms "proximal" and "distal" are to be taken as relative to the user of the delivery devices. "Proximal" is to be understood as relatively close to the user, and "distal" is to be understood as relatively farther away from the user. In other words, the leading end of a delivery device is positioned distal to the trailing end of the delivery device. In the case of an aortic prosthetic heart valve, the "distal" end may be the outflow end and the "proximal" end may be the inflow end.

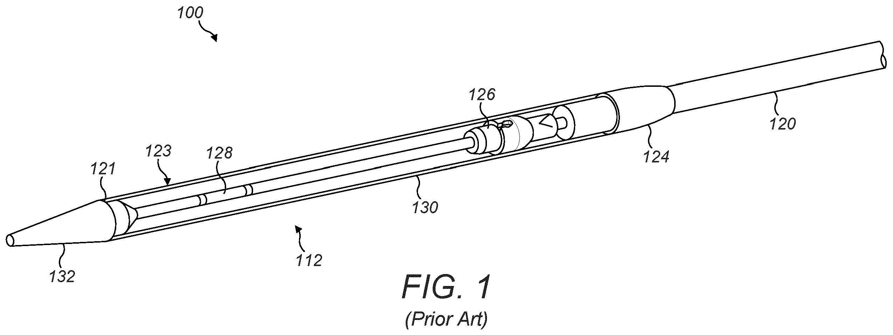

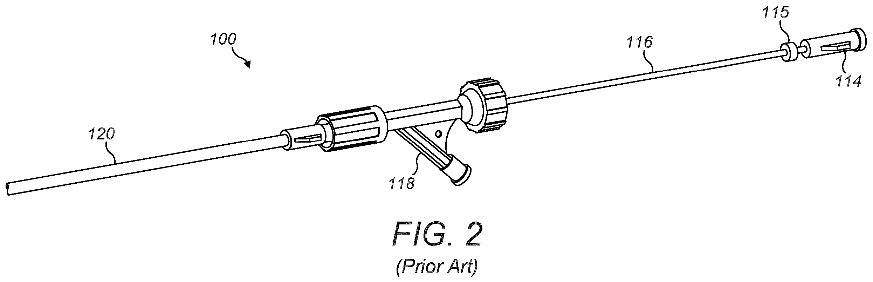

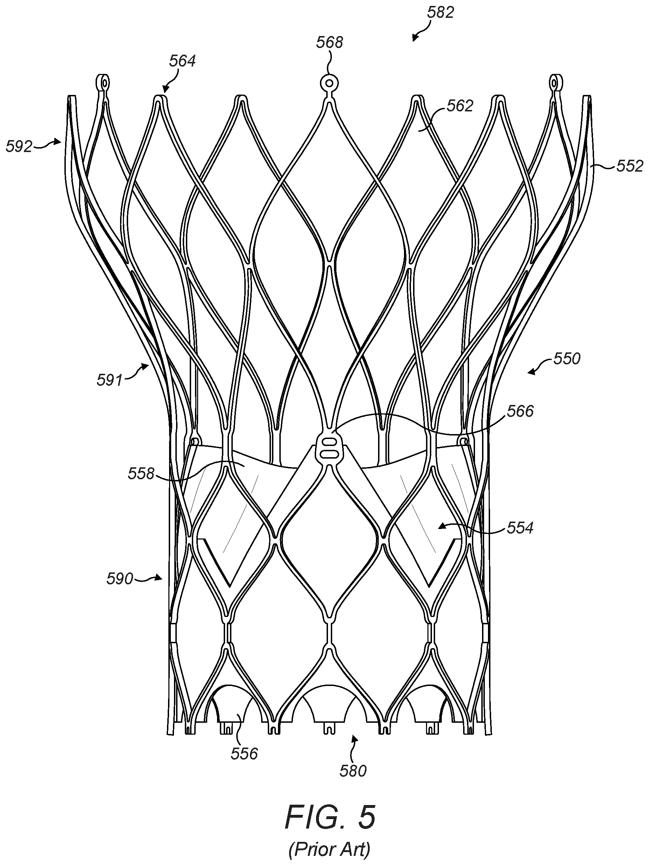

The present disclosure relates to assemblies, devices and methods for loading a self-expanding stent or a collapsible prosthetic heart valve into a minimally invasive delivery device. An exemplary minimally invasive delivery device 100 is illustrated in Figures 1 and 2. The present disclosure also relates to collapsible prosthetic heart valves 550, an example of which is illustrated in Figure 5.

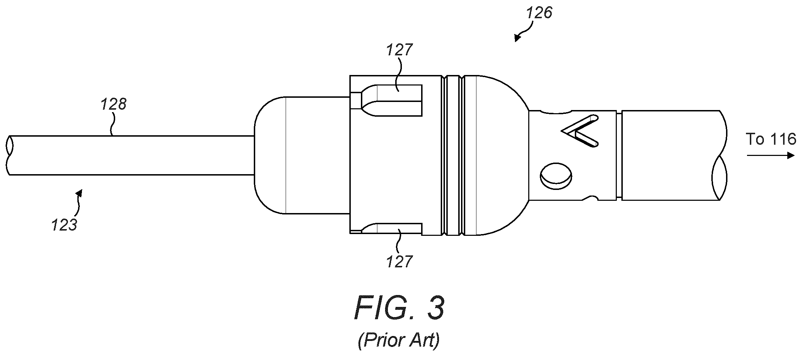

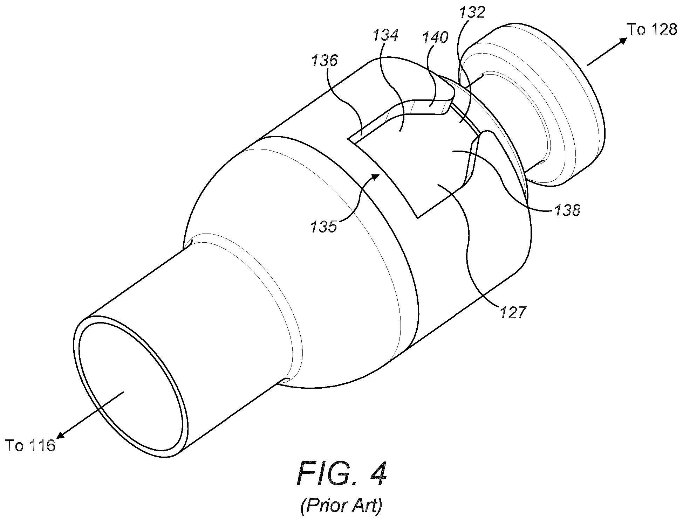

In particular, the present disclosure relates to retainers which secure stents to delivery systems during placement. Stents must remain secured to delivery system during placement but must be completely freed from the delivery system after placement. These retainers may be referred to as pocket-tab type stent retainers in that they typically comprise a pocket or recess on the delivery device into which fits a tab of the stent during loading. An exemplary tab 568 is shown at the outflow end of the prosthetic heart valve 550 illustrated in Figure 5 and an exemplary pocket 127 is shown in Figures 3 and 4. Throughout the present description, the terms pocket and recess will be used interchangeably to refer to the compartment on the delivery device arranged to house the tab. The term tab may also be referred to as a retainer when used in the context of the prosthetic heart valve, but when used in the wider context, the term retainer may refer to the collective pocket-tab arrangement. Similarly, the retaining element of the delivery device may comprise the recess and the retaining element of the stent may comprise the tab.

Exemplary delivery devices for transfemoral delivery of a collapsible prosthetic heart valve (or other types of self-expanding collapsible stents) have a catheter assembly for delivering the heart valve to and deploying the heart valve at a target location. The catheter assembly includes a compartment defined between an atraumatic tip of the delivery device and a retaining element. A support shaft is connected between the tip and the retaining element and defines the length of compartment. A distal sheath is slidably arranged relative to the compartment so that, in a distalmost or closed position in which the distal end of the sheath abuts the atraumatic tip, the sheath covers the prosthetic heart valve and retains it for delivery to the target site, and in a proximal or open position in which the distal end of the sheath is spaced from the atraumatic tip, the sheath uncovers the prosthetic heart valve for deployment at the target site.

The retaining element may include a plurality of recesses located around its periphery. The recesses are spaced apart from one another (e.g. regularly and equally spaced in a circumferential direction) and each is sized and shaped to receive a tab or retainer on one end of the prosthetic heart valve to maintain the prosthetic heart valve in assembled relationship with the delivery device. The stent of the prosthetic heart valve includes this tab or retainer at the outflow end thereof, the retaining elements being sized and shaped to cooperate with the recesses provided on the delivery device.

The cooperating male tab and female recess and the engagement of the male retaining elements with the female retaining structures functions to prevent premature deployment of the prosthetic heart valve from the delivery device, minimise longitudinal movement of the prosthetic heart valve relative to the delivery device during unsheathing and resheathing procedures, help prevent rotation of the prosthetic heart valve relative to the delivery device as the delivery device is advanced to the target site and during deployment, and maintain the alignment of the stent cells and prevent them from becoming tangled. It is important that the stent remain secured to the delivery system during placement but is completely freed from the delivery system after placement.

Improvements are sought to a pocket-tab type stent retainer that can be used to improve the loading of the stent, the securement of the stent to the delivery device and/or its subsequent release.

In specific examples, numerous problems have been identified, at least one or more of which could be resolved or at least mitigated by improvements to the pocket-tab type stent retainer. Firstly, tab-pocket disengagement can be caused by tipping resulting from the weight of the stent and loading tools. Secondly, non-tab stent features can be trapped behind the tab stent features preventing the tab from engaging in the pocket. Similarly, non-tab stent features can be lodged under the retaining element during loading. Lastly, anatomical variables or procedural decisions can impact the release of the tab, otherwise known as tab hang-up. These include but not are limited to: a tight arch, a horizontal aorta, excessive guidewire forward pressure during deployment, a user not fully retracting the capsule, and the orientation of the retainer relative to the plane of the arch.

As seen in Figures 1 and 2, an exemplary delivery device 100 for transfemoral delivery of a collapsible prosthetic heart valve (or other types of self-expanding collapsible stents) has a catheter assembly 112 for delivering the heart valve to and deploying the heart valve at a target location. The catheter assembly 112 includes a compartment 123 defined between an atraumatic tip 132 of the delivery device 100 and a retaining element 126. A support shaft 128 is connected between the tip 132 and the retaining element 126 and defines the length of the compartment 123. A distal sheath 130 is slidably arranged relative to the compartment 123 so that, in a distalmost or closed position in which the distal end 121 of the sheath abuts the atraumatic tip 132, the sheath covers the prosthetic heart valve and retains it for delivery to the target site, and in a proximal or open position in which the distal end 121 of the sheath is spaced from the atraumatic tip 132, the sheath uncovers the prosthetic heart valve for deployment at the target site.

An inner tube 116 having a lumen therethrough extends from a hub 114 at or near its proximal end to a distal end which may be connected to retaining element 126. Optionally, the distal end of inner tube 116 may extend through retaining element 126 and support shaft 128 for connection to atraumatic tip 132. In either arrangement, the distal end of inner tube is connected to the compartment 123 so as to define a fixed distance between the hub 114 and the compartment 123. The lumen through the inner tube 116 is sized to slidingly receive a guidewire (not shown) for use in guiding the delivery device to the target site. At its proximal end, the inner tube 116 may be provided with a hemostasis valve (not shown) for preventing, or at least hindering, blood flow out from the inner tube.

The hub 114 is adapted for connection to another system or mechanism, such as an operating handle (not shown) for displacing the distal sheath 130. Mechanisms for displacing the distal sheath 130 between its proximal and distal positions are described in International Patent Application Publication No. WO/2009/091509, the disclosure of which is hereby incorporated by reference herein. A retaining ring 115 may be mounted on the inner tube 116 near hub 114. Further examples of exemplary delivery devices are described in International Patent Application Publication Nos. WO/2013/016513 and WO/2018/213091, the disclosure of which is hereby incorporated by reference herein. These publications describe systems of loading a collapsible heart valve onto the delivery system and a transcatheter delivery system with wheel actuation relevant to the present disclosure.

The catheter assembly 112 further includes an outer shaft 120 which is connected at its distal end through a tapered transition member 124 to the proximal end of distal sheath 130, and at its proximal end to the operating handle (not shown). A Y-connector 118 may also be connected at the proximal end of outer shaft 120 and may include a hemostasis valve for hindering blood flow out from between the inner tube 116 and the outer shaft 120. The Y-connector 118 may also be coupled to a fluid source for flushing the outer shaft 120, injecting contrast media during a prosthetic valve implantation procedure, and the like.

It should be noted that the exemplary delivery device is presented for context but any suitable delivery device comprising retainers may be utilised with the principles of the present disclosure.

As shown in Figure 3, the retaining element 126 may include a plurality of recesses 127 located around its periphery. The recesses 127 are spaced apart from one another (e.g. regularly spaced in a circumferential direction) and each is sized and shaped to receive a tab or retainer on one end of the prosthetic heart valve to maintain the prosthetic heart valve in assembled relationship with the delivery device 100.

Although illustrated as being connected to the inner tube 116 at its proximal end and support shaft 128 at its distal end, it will be understood that any suitable coupling of the retaining element 126 to the delivery system may be utilised, with the retaining element 126 preferably defining the proximal end of the compartment 123.

Figure 4 illustrates the retaining element 126 from a different perspective. As shown, the recess 127 of the retaining element may comprise a neck opening 132 or narrow channel opening into which sits a tab connecting portion of the prosthetic heart valve, e.g. a corresponding neck of the tab. The opening expands into a wider chamber 134 arranged to receive the tab. The recess 127 comprises a rear wall 135, side walls 136 and a base 138 and may be any suitable shape to receive and retain the tab. The exemplary shape may alternatively be considered to be trapezoidal having straight sides with a wider chamber narrowing towards the opening. Put another way, the recess may be tapered and may have a bottleneck shape, similar to a pointed arch when viewed from the proximal end, with two side walls 136 perpendicular to the rear wall 135 and each side wall 136 comprising a narrowing portion 140 narrowing towards the opening 132. The base 138 may also be thought of as a pad, floor or bottom surface of the recess.

In use, the tab may abut any of the walls during loading and delivery, for example axial movement of the stent may cause the tab to engage the rear, or proximal, wall 135 or the two narrowing, distal, side wall portions 140. During circumferential movement, the tab may abut either or both of the two perpendicular sidewalls 136.

During loading of a collapsible heart valve, a tab 568 is placed into the recess to secure the stent onto the delivery device. An exemplary tab 568 is illustrated in Figure 5 together with an exemplary collapsible heart valve 550 of which it is a part.

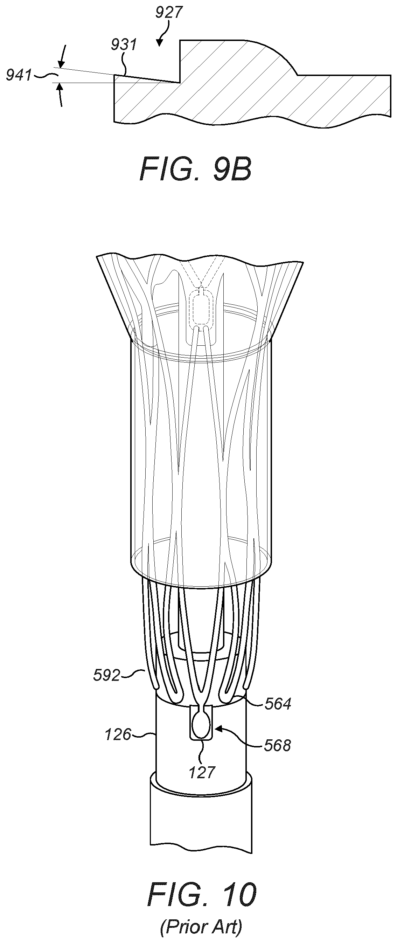

The prosthetic heart valve 550 is designed to replace the function of a native aortic valve of a patient. Examples of collapsible prosthetic heart valves are described in International Patent Application Publication No. WO/2009/042196; and U.S. Pat. Nos. 7,018,406 and 7,329,278, the disclosures of all of which are hereby incorporated herein by reference. As discussed in detail below, the prosthetic heart valve has an expanded condition, shown in Figure 5, and a collapsed condition. A portion of the prosthetic heart valve 550 is shown during a stage of loading in Figure 10. Although the delivery system is described herein in connection with its use to deliver a prosthetic heart valve for replacing a native aortic valve, the delivery system is not so limited, and may be used to deliver prosthetic valves for replacing other types of native or prosthetic cardiac valves.

The prosthetic heart valve 550 includes an expandable stent 552 which may be formed from any biocompatible material, such as metals, synthetic polymers or biopolymers capable of functioning as a stent. The stent 552 extends from an inflow or proximal or annulus end 580 to a distal or outflow or aortic end 582, and includes an annulus section 590 adjacent the inflow end and an aortic section 592 adjacent the outflow end. The annulus section 590 has a relatively small cross-section in the expanded condition, while the aortic section 592 has a relatively large cross-section in the expanded condition. Preferably, the annulus section 590 is in the form of a cylinder having a substantially constant diameter along its length. A transition section 591 may taper outwardly from the annulus section 590 to the aortic section 592. Each of the sections of the stent 552 includes a plurality of cells 562 connected to one another in one or more annular rows around the stent. For example, as shown in Figure 5, the annulus section 590 may have two annular rows of complete cells 562 and the aortic section 592 and transition section 591 may each have one or more annular rows of partial cells 562. The cells 562 in the aortic section 592 may be larger than the cells 562 in the annulus section 590. The larger cells in the aortic section 592 better enable the prosthetic valve 550 to be positioned without the stent structure interfering with blood flow to the coronary arteries.

The stent 552 may include one or more retaining elements, or tabs, 568 at the outflow end 582 thereof, which are the subject of the present disclosure; the retaining elements being sized and shaped to cooperate with female retaining structures provided on the delivery device. The engagement of tabs 568 with the female retaining structures on the delivery device helps maintain prosthetic heart valve 550 in assembled relationship with the delivery device, minimises longitudinal movement of the prosthetic heart valve relative to the delivery device during unsheathing or resheathing procedures, and helps prevent rotation of the prosthetic heart valve relative to the delivery device as the delivery device is advanced to the target location and during deployment.

While the tab 568 is illustrated as having a round shape and engaging in the tapered recess 127, it may be understood that the tab 568 and recess 127 may be any suitable shape arranged to prevent longitudinal movement and/or rotation of the stent when loaded onto the device.

In between the tabs 568 at the outflow end 582 are junctions of the struts of the cells 562 in the end row of cells (for an aortic valve the distalmost row of cells). Each pair of struts meet at a respective apex 564, these being collectively referred to as apices at the outflow end (i.e. the aortic stent apices or the distal end for an aortic valve). While Figure 5 illustrates three tabs, each separated by two apices so that there are collectively six apices, it will be understood that the stent may comprise any quantity, sequence or arrangement of tabs and apices.

The prosthetic heart valve 550 includes a valve assembly 554 positioned in the annulus section 590. Valve assembly 564 includes a cuff 556 and a plurality of leaflets 558 which collectively function as a one-way valve. The commissure between adjacent leaflets 558 may be connected to commissure features 566 on stent 552. Figure 5 illustrates a prosthetic heart valve for replacing a native tricuspid valve, such as the aortic valve. Accordingly, prosthetic heart valve 500 is shown in Figure 5 with three leaflets 558, as well as three commissure features 566. As can be seen in Figure 5, the commissure features 566 may lie at the intersection of four cells 562, two of the cells being adjacent one another in the same annular row, and the other two cells being in different annular rows and lying in end-to-end relationship. Preferably, commissure features 566 are positioned entirely within annulus section 590 or at the juncture of annulus section 590 and transition section 591. Commissure features 566 may include one or more eyelets which facilitate the suturing of the leaflet commissure to the stent. However, it will be appreciated that the prosthetic heart valves may have a greater or fewer number of leaflets and commissure features. Additionally, although cuff 566 is shown in Figure 5 as being disposed on the luminal surface of annulus section 590, it is contemplated that the cuff may be disposed on the abluminal surface of annulus section 590, or may cover all or part of either or both of the luminal and abluminal surfaces of annulus section 590. Both the cuff 556 and the leaflets 558 may be wholly or partly formed of any suitable biological material or polymer.

In operation, a prosthetic heart valve, including the prosthetic heart valve described above, may be used to replace a native heart valve, such as the aortic valve, a surgical heart valve or a heart valve that has undergone a surgical procedure. The prosthetic heart valve may be delivered to the desired site (e.g., near a native aortic annulus) using any suitable delivery device, including the delivery devices described in detail below. During delivery, the prosthetic heart valve is disposed inside the delivery device in the collapsed condition. The delivery device may be introduced into a patient using a transfemoral approach, transapical approach, a transseptal approach, or any other suitable approach. Once the delivery device has reached the target site, the user may deploy the prosthetic heart valve. Upon deployment, the prosthetic heart valve expands into secure engagement within the native aortic annulus. When the prosthetic heart valve is properly positioned inside the heart, it works as a one-way valve, allowing blood to flow in one direction and preventing blood from flowing in the opposite direction.

In a prosthetic heart valve, the valve assembly may be spaced from the outflow or aortic end of the stent by a distance that enables deployment of the heart valve by an amount sufficient for the valve leaflets of the prosthetic valve to operate as intended, while the outflow end of the stent remains captured by the delivery device. More particularly, as will be explained further below, the annulus end of the prosthetic heart valve may be deployed first, while the aortic end of the prosthetic heart valve remains at least partially covered by a distal sheath of the delivery device. The annulus portion of the prosthetic heart valve may be deployed so that the entirety of the valve leaflets, up to and including the commissures, is deployed and fully operational. By deploying the prosthetic heart valve in this manner, the user can determine whether the valve leaflets are properly positioned relative to the native valve annulus, and whether the valve is functioning properly. If the user determines that the positioning and operation of the valve are acceptable, the remainder of the valve may be deployed. However, if it is determined that the leaflet position is improper or that the valve is not functioning properly, the user may resheath the valve and either reposition it for redeployment, or remove it entirely from the patient. This can be particularly important in very high risk patients who would typically be recipients of these types of valves because of the nature of their condition and the impact that may have on the shape and/or condition of the native valve and valve annulus.

As is shown in Figure 5, in one example the entirety of valve assembly 554, including the leaflet commissures, is positioned in the annulus section 590 of stent 552. When opened, the leaflets may extend further into the transition section 591 or may be designed such that they remain substantially completely within the annulus section. That is, substantially the entirety of valve assembly 554 is positioned between the inflow end 580 of stent 552 and the commissure features 566, and none of the valve assembly 554 is positioned between commissure features 566 and the outflow end 582 of the stent. Indeed, in some embodiments, the valve can be designed such that, upon partial deployment, the commissure features are fully exposed, oriented generally parallel to the direction of blood flow, and at or near their actual radially expanded positions (but not necessarily their eventual positions relative to the annulus), such that the leaflets can operate substantially as they would when the valve is fully deployed, even though enough of the stent is still retained within the delivery device or sheath to permit resheathing.

Improvements are sought to improve the loading, securement, and release of the tabs 568 from their corresponding recesses 127. For example, during the securement process, when tabs 568 are positioned on the retainer 126, they must remain in the pockets 127 until fully encapsulated by delivery system. If one or more of the tabs 568 pop out of pockets, misload can occur. Further, stent and loading tools can cause the system to be top heavy, and tipping can lead to tab-pocket disengagement. Moreover, non-tab stent features (such as apices 564) can get trapped behind tab stent features (referred to as strut crossover) preventing the tab 568 from engaging in the pocket 127. In further examples, during deployment and release of the valve, once the stent is deployed, it must be completely freed from the delivery system. If the tabs 568 do not fully release, the delivery system cannot be safely withdrawn without affecting the position of the stent and potentially harming the patient.

Various concepts are presented with may be utilised individually or in combination to improve securement and release of the tabs. In other words, the following describes improvements to a pocket-tab type stent retainer that can be used solo or in concert to improve securement and/or release.

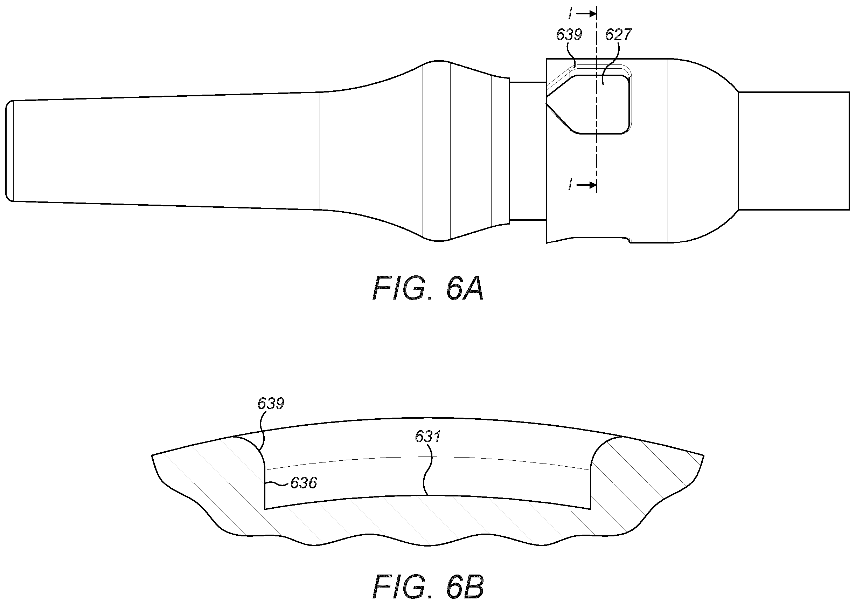

In one contemplated example, the recess may be adapted or otherwise machined to create a certain profile while reduces friction on the tab during release while ensuring securement of the stent during delivery. Examples are illustrated in Figures 6, 7 and 9. By release we mean the expansion of the prosthetic heart valve from its collapsed condition to its expanded condition. In other words, the recess is shaped or adapted to reduce contact with the tab (e.g. progressively reduce) as the prosthetic heart valve expands.

Thus, one or more of the dimensions of the recess may increase from a base of the recess to the outer surface of the retaining element in which the recess is formed. Hence, the recess may comprise a larger length parallel to the insertion direction (distally to proximally) or a larger width perpendicular to the insertion direction at the surface of the retaining element than at the base of the recess.

These examples address the specific problem that, once the stent is deployed, it must be completely freed from the delivery system. If tabs do not fully release, the delivery system cannot be safely withdrawn without affecting the position of the stent and potentially harming the patient. Some anatomical variables or procedural decisions can impact tab hang-up including but not limited to: tight arch, horizontal aorta, excessive guidewire forward pressure during deployment, not fully retracting the capsule, and orientation of the retainer relative to the plane of the arch.

Figures 6A and 6B illustrate a first example in which one or more of the top edges 639 of the recess 627 may be radiused. Figure 6A shows a perspective view of the radiused top edge while Figure 6B illustrates a cross section of the recess showing the curvature of the edge 639. In this example, 'top' refers to the direction radially outward from a central longitudinal axis of the delivery device. Figure 6B shows the radiused top edge 639 extending from the side wall 636 which meets the base 631. The edge may thus be thought of as rolled.

In this example, a radius is added along the outermost edge of the recess (in other words the 'top' edge) or retainer pocket to remove material that impedes tab movement out of the recess of pocket. Preferably the entire outermost edge may be radiused, but it will be understood that benefits may still arise from only one or more of the edges being recessed in order to create a certain friction profile. For example, it may be beneficial to maintain a degree of hold by not rolling the rear wall, that is, the top or outermost edge of the rear wall.

The radius in effect reduces the surface area contact, and therefore friction, between the tab 568 and the device as the tab releases. The extent of curvature may be a balance of retention against release profile, for example, the degree of curvature and the proximity of the curvature to the base may accelerate or retard the relative release of the tab from the recess while potentially affecting the retention of the stent in the retaining element.

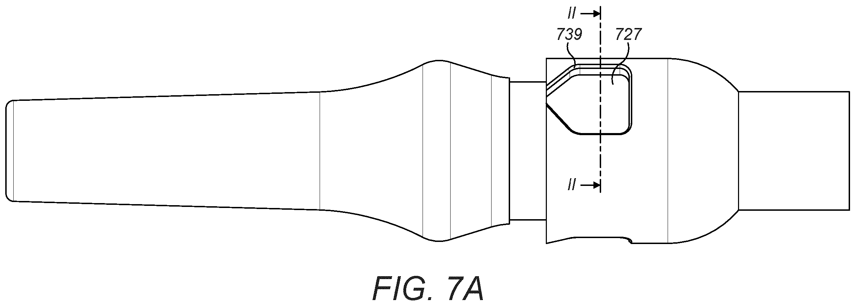

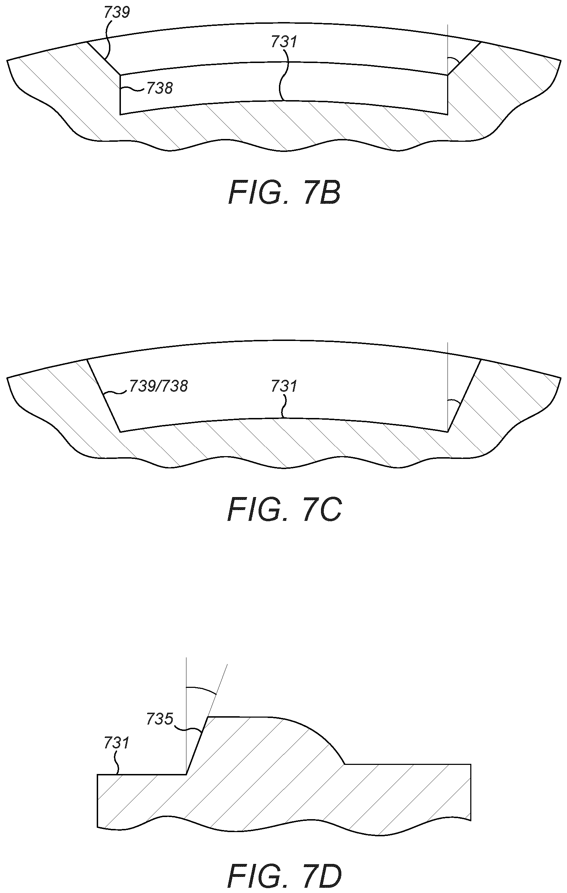

In an alternative example, shown in Figures 7A and 7B, one or more of the 'top' or outermost edges 739 of the recess 727 may be drafted. Again, in this example, 'top' refers to the direction radially outward from a central longitudinal axis of the delivery device. As shown in Figure 7B, the outer edge may be tapered at an angle away from the plane of the side wall 738. As with Figures 6A and 6B, the degree of taper may be adjusted to obtain a particular release profile. In other words, in this example, a chamfer is added along the entire outermost edge of the retainer pocket to remove material that impedes tab movement out of the pocket.

Alternatively, the full height of the walls 738 of the pocket could be drafted. Such an example is shown in Figure 7C. In this example, the side wall 738 extends at an angle away from the base. The angle may be selected to ensure retention of the tab while still facilitating release. Any combination of taper and radius is contemplated, for example, the side wall 738 may be tapered and its outermost edge radiused.

This tapered outer edge may also be thought of as a bevel surrounding the recess. As with Figures 6A and 6B, in the example of Figures 7A, 7B and 7C, any number of edges may be tapered. In the examples of Figures 6A, 6B, 7A, 7B and 7C, preferably at least the edges of the narrowing side wall portions that extend from the perpendicular side wall to the neck opening may be tapered. These edges are anticipated to have the most direct impact on the release of the tabs, in use.

Since all the outermost edges may be tapered, this includes that the rear wall (proximal wall) 735 may also be tapered. In practice, this may be profiled differently to the side walls. An example is shown in Figure 7D. A tapered rear wall 735 may be used together with any combination of profiled side walls. Note, in use, the rear or proximal wall 735 may serve to prevent axial movement of the stent and so a degree of tapering of this wall may result in more or less axial movement of the stent and an increase in load borne by different components of the stent on the compartment. In one example, the rear wall may not need to be tapered since the distal end of the tab may not abut the rear wall if the recess is enlarged. As will be described below, alternative means may be necessitated to control axial movement of the stent.

Where profiled edges are referred to it is contemplated that this term may encompass edges that have been given a particular shape or cross-sectional profile, which could include: rounded edges (e.g. radiused); bevelled edges; stepped edges; chamfered edges; and other curved profiles.

In the previous example it was described how the walls of the recess may be profiled to achieve a particular release profile. In an additional combinable option, the base 931 of the recess 927 may be tapered from the opening to the rear wall, in other words tapered distally to proximally. Thus, the depth of the recess 927 may increase from the opening of the recess 927 to the rear wall.

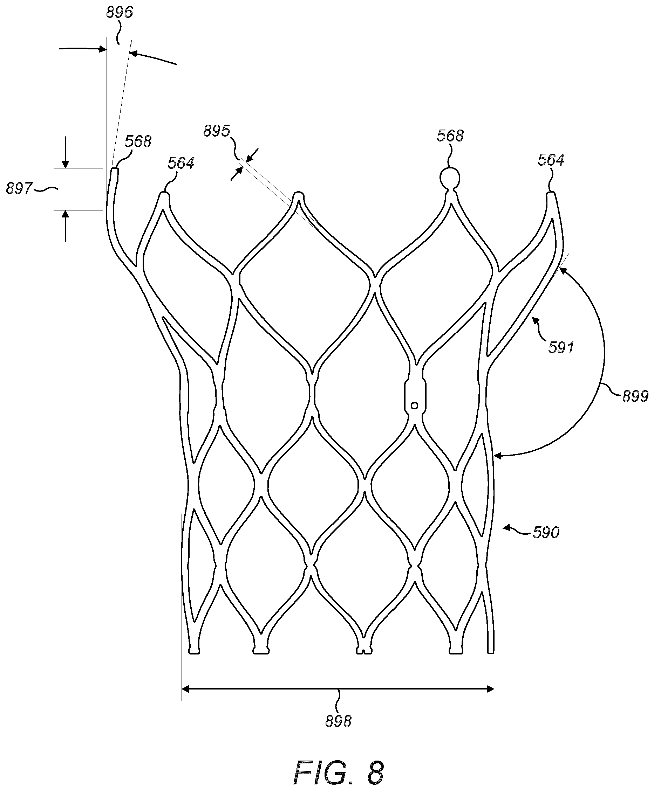

For context, an exemplary heart valve stent is shown in Figure 8. In contemporary collapsible heart valves, the outflow end row of struts of the stent may be formed at an angle relative to the axis of the stent. An exemplary angle 896 shown in Figure 8 may be 10°. This is of course merely exemplary. In other words, the tabs and optionally the apices are directed inwardly at a 10° angle relative to the axis of the stent, that is, to a line parallel with the central longitudinal axis. A vertical distance 897 between the vertical tangent point to the top edge of the stent may preferably be 0.157" (4mm).

Figure 8 also shows an angle 899 of 146° between the outer diameter of the annulus section 590, e.g. an axis parallel to the central longitudinal axis of the stent, and the struts of the transition section 591; that is, 34° from vertical.

The exemplary stent of Figure 8 has an annulus outer diameter of 31.5mm ± 0.3mm and an aortic strut width of 0.124mm ± 0.0013mm.

In short, the illustrative stent of Figure 8 shows that the outflow end row of struts, and as a consequence the apices and/or tabs, may be angled inwardly.

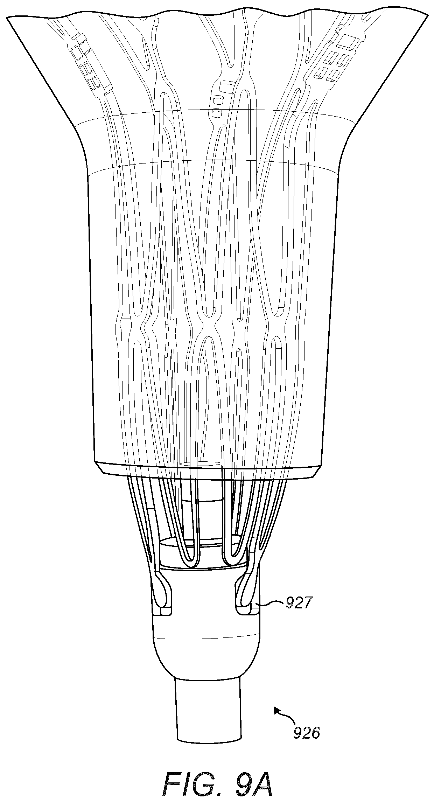

Figure 9A illustrates an additional combinable example of a retaining element 926 in which the base 931 or inner bottom surface is tapered to substantially match the inward angle of the tab 568. Put another way, the floor of the recess may be raked. In this specific example, the taper angle could be 10° from the horizontal plane, i.e. 80° from the rear, or proximal, wall. In other words, the flat or rounded base of the retainer pocket is angled to match the angle of the stent tab; that is, there is a general reduction in outer diameter of the retaining element in the recess in the distal to proximal direction, preferably substantially corresponding to a reduction in outer diameter of the stent of the prosthetic heart valve in an inflow to outflow direction.

It will be understood that this taper angle may not be substantially the same as the angle of the tab but an angled base, even if not having substantially matching angle, may still provide the noted advantages. These advantages being that the tab is enabled to sit deeper in the pocket and the change reduces the amount of open space underneath the tabbed stent struts which helps prevent non-tabbed struts, e.g. apices 564, from passing underneath the stent and causing misload. The angle of the taper in the example shown in Figures 9A and B is 7°. Preferably the angle of taper is configured to be between 0° and 20°, more preferably between 7° and 10°.

Additionally, the taper reduces stress on the tabs caused by the collapsed valve secured on the retaining element and importantly helps to prevent tab-pocket disengagement caused by tipping, where outward force on the tabs by the retaining element combines with top heavy stent and loading tools to push the tab out of the pocket. The taper of the bottom surface helps to reduce the outward force on the tab during loading and helps to prevent misalignment.

Figure 9B illustrates a cross-sectional view of the retaining element and recess 927 showing the tapered profile of the base. The angle 941 of taper from the distal end to the proximal end can be any suitable taper to alleviate force on the tabs, however in this example the angle substantially matches that of the inward angles of the tabs -in this specific example it is 7-10°. As will be seen, the depth of the recess 927 increases from the opening on the left-hand side of Figure 9B to the rear wall.

The above-described adaptations are made to the one or more recesses of the retaining element arranged to receive the one or more tabs of the stent. These recesses may be referred to as primary recesses. In the following, there is described one or more secondary recesses arranged to receive non-tab features of the stent, such as the apices of the outflow-most or distalmost row of struts of the stent. In other words, additional pocket features for stent aortic apices may be added to the retainer to prevent strut crossover.

For context, a contemporary collapsible prosthetic heart valved loaded onto the retaining element is illustrated in Figure 10. Figure 10 shows a tab 568, e.g. a round piece of stent engaging with a retainer pocket 127. The apices of the struts of the outflow-most or distalmost row of cells, e.g. in the aortic section 592, are shown as abutting the retaining element 126. By distalmost here we refer to the outflow end apices for aortic valves.

In this arrangement, non-tab stent features can get trapped behind tab stent features (referred to as strut crossover) preventing the tab from engaging in the pocket or alternatively can be caused to splay, with similar outcomes; that is, the nitinol of the struts is under significant pressure and wants to splay out or deflect inwards. Similarly, the non-tab stent features may be load bearing against the retaining element. This may be beneficial to prevent axial movement but is also preferably controlled to avoid misalignment and stent damage.

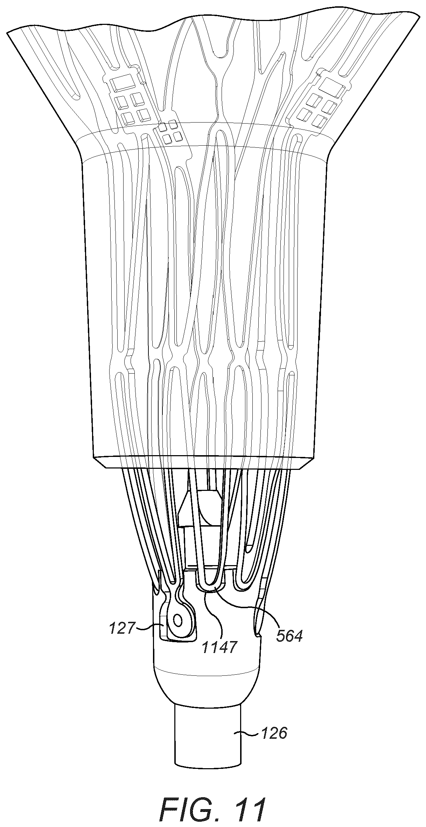

Figure 11 illustrates secondary recesses 1147 each receiving a respective apex 564 of the struts of the stent. In preferred examples the secondary recesses may be indentations in the retaining element sized and configured to receive the apices, however these may also have the characteristics described above with respect to the primary recesses 127; for example, the indentations may have a tapered base.

To accommodate the secondary recesses, the outer diameter of the retaining element may extend further in a distal direction, i.e. in a direction away from the rear wall of the primary recesses, to accommodate the secondary recesses. Alternatively, the primary recesses may be made larger to accommodate the tabs being received in the retaining element further in the proximal direction.

In short, additional pocket features for stent aortic apices may be added to the retainer to prevent strut crossover. During loading, each of the aortic stent apices are positioned in an individual pocket. These pockets are spaced out (e.g. regularly spaced in a circumferential direction) around the diameter of the retainer and prevent the non-tabbed aortic struts from crossing over or under one another. If one of these stent struts crosses underneath a tabbed stent strut, it can prevent the tabbed stent strut from fully engaging with the retainer resulting in misload. The secondary recesses also help to prevent circumferential sliding and also contribute to preventing axial movement depending on the size and configuration of the primary and secondary recesses. With certain shapes and configurations, the secondary recesses may also alleviate axial load on the struts caused where, with previous configurations, the struts may abut the distal end of the retaining element.

In this regard, it is noted that the size and configuration of the primary and secondary recesses may result in each working together to prevent or increase axial and circumferential loading. For example, a larger primary recess in the axial direction may refer axial load to the apices since there will be reduced contact between the tabs and the rear wall of the recess. Similarly, a larger indentation of the secondary recess in the axial direction will alleviate axial (i.e. longitudinal) load on the apices while moving that load to the tabs. For an apices-loaded scenario, primary recesses may preferably be 0.10" (2.54mm) or greater and secondary recesses may preferably be 0.04" (1.016mm) or less. For a tab-loaded scenario, primary recesses may preferably be 0.098" (2.4892mm) or less and secondary recesses may preferably be greater than 0.04" (1.016mm).

In a further contemplated example, the outer diameter of the retaining element extends away from the primary recesses with a narrower outer diameter than the outer diameter of the recesses, thereby providing a stepped profile to prevent inward crossover of the non-tabbed struts of the outflow end. This example may be combined with the indentations, i.e. the narrower outer diameter may further comprise a secondary recess to receive the apices.

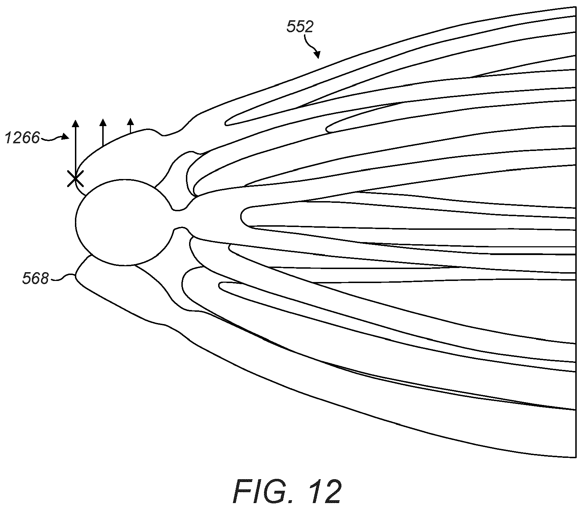

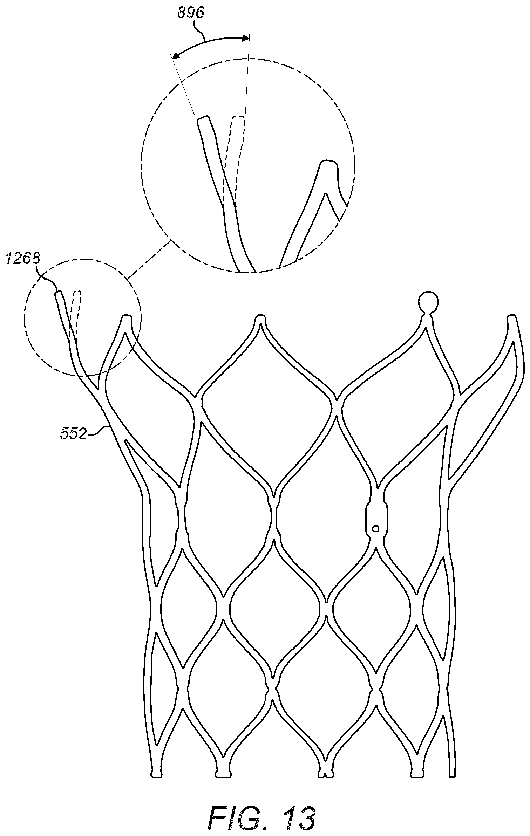

The above concepts were concerned with adaptations to a retaining element of an exemplary delivery device. However, it is also contemplated that adaptations may be made to the features of the stent so as to achieve similar technical benefits, either working together with the above-described adaptations to the retaining element or in isolation. Figures 12 and 13 illustrate one such exemplary adaptation to the stent.

In this example, stent tab orientation is modified to be aligned with the axis of the retainer, i.e. the delivery system, rather than following the natural curvature of the aortic cells. Referring back to Figure 8, it was illustrated that the struts of the stent may each follow a different curvature relative to one another. For example, the outflow struts and tabs may be inwardly angled at approximately 10° to the axis of the stent. Figure 12 illustrates this conceptually, with Figure 13 illustrating an alternative cross section of the same alternative tab angle. Figure 12 shows the tabs 568 at the end of the struts in the aortic section 592. The tabs 568 extend at substantially the same angle as the struts. The arrows 1266 of Figure 12 illustrate conceptually how the angle of the tabs may be set outwardly, back against the direction of curvature. Figure 13 illustrates an angled tab 1268 shaped in an outward direction relative to the struts of the outflow row of cells. As will be seen from Figure 13, an angle 896 exists between the direction in which the angled tab 1268 extends and the direction in which the struts of the outflow row of cells extend.

The angle may be formed by setting the shape (e.g. via heat treatment) of the valve tab relative to other portions of the stent.

It will be noted that a goal is for the tabs to be parallel to the central axis of the valve when in its constrained loading state. Practically, therefore the tabs may be tapered out, leading to an angle of approximately 30° from their orientation before tapering, i.e. the plane of the struts supporting the tabs).

Preferably the valve tab shape may be adapted to be axially aligned with the retaining element when the valve is collapsed and mounted on the retaining element, instead of being tapered inwardly.

Above it was described that the base of the recess on the retaining element may be tapered to match the taper of the tabs. This example is therefore an alternative, but this example may also work together with that above-described concept. For example, the recess may be tapered between 0 and 10° with the tab also angled outwardly with respect to the curvature of the strut between 0 and 10° to substantially correspond to the tapered recess. Although, as above, the angles may not exactly match and may be similar to achieve the same technical advantages.

In summary, stent tab orientation may be modified to be aligned with the axis of the retaining element when the stent is mounted on the retaining element rather than following the natural curvature of the aortic cells. This change reduces the amount that the tabs are radially driven into the retaining element by the set shape of the Nitinol stent. This change also causes the tab to lie flat in the retainer pocket which reduces the amount of open space under the tabbed stent struts which helps prevent non-tabbed struts from passing underneath it and causing misload. Other advantages similar to those described in the context of the tapered recess example also apply to this example. That is, among others, outward force on the tabs from the retaining element are reduced, preventing misalignment.

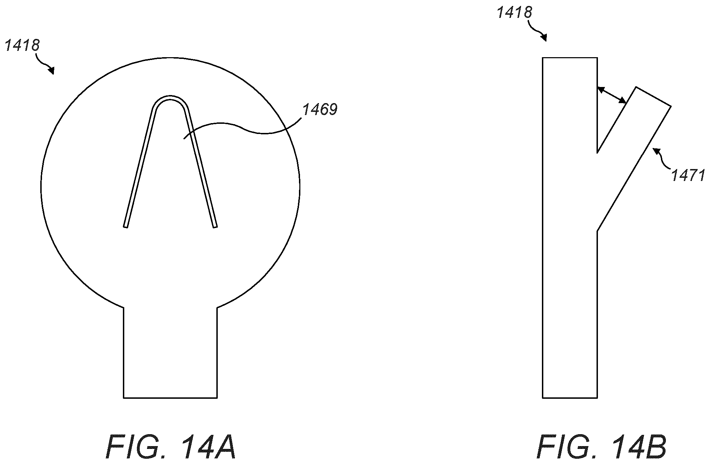

A further adaptation to the tabs will now be described in the context of Figures 14A and 14B. In this example a tab of the stent comprises a biasing element arranged to bias the tab outwardly with respect to the recess. In a specific example, a spring feature is cut and shape-set (e.g. via heat treatment) into the Nitinol tab of the stent that causes it to push itself away from the retainer when released.

Figures 14A and B illustrate such an example where the tab 1418 comprises a cut out or perforation 1469. A spring-loaded feature 1471 or flap is then shape set into the tab. The spring feature is arranged on the inner side of the tab, i.e. is direct inwardly of the stent, so that it is configured to bias the tab away from the recess. In implementation, during release, the feature pushes the tab off the retainer when fully released. The spring feature may be shape set at an angle between 0° and 45° relative to the plane of the tab, preferably between 20° and 40°.

The cut out 1469 may preferably be in the form of an inverted 'v'-shape or a chevron, as shown, extending from its widest point at the inflow end of the tab, i.e. its base, to its narrowest point at the outflow end, i.e. from bottom to top or from the inflow end to the outflow end, optionally with a generally acute angle at its peak. Its narrowest point may be shape set to be biased away (e.g. radially inwardly) from the tab. This geometry is generally advantageous as it facilitates movement of the spring; however, even more advantageously, this geometry does not interrupt blood flow and is therefore beneficial when implanted in the human anatomy.

Other geometries are of course contemplated such as a generic shape extending from the inflow end to the outflow end; as well as an 'n' shape, a rectangular geometry, a circular cut out (e.g. cut from 7 o'clock to 4 o'clock on a clock, in other words an arc, or major arc, covering approximately 275° of a circle) or one or more strips. A spiral cut out resembling a traditional helical coil shape may also be used. In a further example the cut out or the flap may be arranged down one side of the tab. In each of these contemplated geometries it may be preferable for the flap to be extending from a base at the inflow end to the outflow end away from the tab so as not to impede fluid flow through the valve.

The biasing element may be biased so as to facilitate push away from the retaining element but not so strongly biased as to impede or prevent assembly, e.g. loading of the stent onto the retaining element. At the same time, the tab as a whole must remain strong enough to function as a tab and to withstand the various forces imparted on it during delivery.

Preferably the feature is, or is configured to be, visible under fluoroscopy to confirm tab placement. This visibility may be a result of the geometric pattern of the cut out. When the tab is loaded correctly, the bias element 1471 may not be separately visible from the rest of the tab 1418. As such, tab placement can be confirmed by checking for visibility of the bias element. The contrary may also be true, that is, if the valve is misloaded, the bias element would still be visible (due to extra space between the tab and the bottom of the pocket).

In summary, a spring feature may be cut and shape-set into the Nitinol tab of the stent that causes it to push itself away from the retainer when released. In this example, a geometric feature may be cut into the nitinol of the tab and shape set to deflect inward relative to the plane of the tab. This inwardly deflected portion acts as a spring to bias the tab away from the base of the retainer pocket. The spring is compressed by the loading system during encapsulation, then pushes the tab out of the retainer pocket as soon as the tab is freed from the capsule, improving release.

In the various concepts above, it has been described how individually and collectively a recess in a retaining element of a delivery device and a corresponding tab of a collapsible prosthetic heart valve may be adapted to balance the conflicting objectives of maintaining securement and facilitating release, while ensuring effective loading and preventing misloads. In a specific example of these concepts functioning collectively, an inverted 'v' shape perforation may be cut into a tab of a stent and a shape set feature configured to point inwardly to form a biasing element arranged to bias the tab outwardly when the tab is secure on a retaining element of the delivery device. The tab may itself be shape set at an angle away from the plane of the struts of the stent so as to align with an axis of the retaining element. A recess on the retaining element into which the tab sits may have its edge a chamfered so as to improve release of the tab. A set of indentations may be made in an extended portion of the retaining element arranged to receive the non-tab apices of the struts of the outflow end. As demonstrated, it is envisaged that each concept described may be used in isolation or together with any of the other contemplated designs to achieve synergistic technical advantages in that each adaptation balances the requirements of securement and release.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A delivery device for a prosthetic heart valve, the delivery device comprising:
an inner shaft;
a distal sheath disposed about a portion of the inner shaft to form a compartment with the inner shaft, the compartment being sized to receive the prosthetic heart valve, the inner shaft and the distal sheath being movable relative to one another; and,
a retaining element coupled to the inner shaft and defining an end of the compartment, the retaining element comprising a recess arranged to receive a corresponding tab of the prosthetic heart valve,
wherein the recess is shaped to progressively reduce contact with the respective tab as the prosthetic heart valve expands from its collapsed condition to its expanded condition.

2. A delivery device according to claim 1, wherein the one or more outer edges of the recess are profiled so that the one or more outer edges are angled outward.

3. A delivery device according to claim 1 or 2, wherein the recess comprises one or more radiused outer edges.

4. A delivery device according to claim 3, wherein the recess comprises a distal opening, two opposing side walls and a proximal wall, the side walls comprising a straight portion and a distal neck portion, wherein the one or more radiused outer edges are the edges of the distal neck portion.

5. A delivery device according to claim 3 or 4, wherein the entire outer edge of the recess is radiused.

6. A delivery device according to any preceding claim, wherein the recess comprises one or more tapered outer edges.

7. A delivery device according to any preceding claim, wherein the recess comprises one or more tapered walls.

8. A delivery device according to claim 7, wherein the recess comprises a distal opening, two opposing side walls and a proximal wall, the side walls comprising a straight portion and a distal neck portion, wherein the distal neck portion is tapered.

9. A delivery device according to any preceding claim, wherein the recess comprises a tapered proximal wall.

10. A delivery device according to any preceding claim, wherein the recess comprises a tapered base.

11. A delivery device according to claim 10, wherein the tapered base comprises a taper extending from a distal opening of the recess adjacent the prosthetic heart valve when the prosthetic heart valve is secured on the retaining element to a proximal wall of the recess remote from the prosthetic heart valve when the prosthetic heart valve is secured on the retaining element.

12. A delivery device according to claim 10 or 11, wherein the tapered base comprises a taper configured to have an angle of taper corresponding to an angle of the tab of the prosthetic heart valve relative to a longitudinal axis of the prosthetic heart valve.

13. A delivery device according to claim 12, wherein the angle is between 0 degrees and 20 degrees.

14. A delivery device according to claim 13, wherein the angle is between 7 degrees and 10 degrees.

15. A delivery device according to any preceding claim, wherein the recess is a primary recess and the retaining element further comprises one or more secondary recesses arranged to receive one or more struts of the prosthetic heart valve.
